# EUROPEAN PATENT SPECIFICATION

(11) **EP 1 418 971 B1**
(45) Date of publication and mention of the grant of the patent: **30.05.2007**
(21) Application number: 02804383.4
(22) Date of filing: 31.07.2002
(51) Int. Cl.: A61M 25/00

(54) **FLOW-DIRECTED CATHETER GUIDE WITH VARIABLE RIGIDITY SHAFT**
KATHETERFÜHRUNG IN FLUSSRICHTUNG MIT SCHAFT VARIABLER STEIFHEIT
GUIDE POUR CATHETER FLOTTANT COMPRENANT UNE TIGE A RIGIDITE VARIABLE

(30) Priority: 31.07.2001 US 309268 P
(43) Date of publication of application: 19.05.2004
(73) Proprietor: Belson, Amir, Cupertino, CA 95014 (US)
(72) Inventor: Belson, Amir, Cupertino, CA 95014 (US)
(74) Representative: Thévenet, Jean-Bruno
(86) International application number: PCT/US2002/024347
(87) International publication number: WO 2003/047675

(56) References cited:
- EP-A- 0 306 010
- WO-A-97/44086
- US-A- 3 995 623

## Description

### FIELD OF THE INVENTION

The present invention relates generally to catheters and catheter guides. More particularly, it relates to a flow-directed catheter guide with a variable rigidity shaft to assist in insertion and guidance of a vascular catheter. A catheter guide according to the preamble of claim 1 is known for example from US-A-3 995 623.

### BACKGROUND OF THE INVENTION

The Seldinger technique is a well-known method for percutaneous insertion of catheters into a patient's blood vessels. Typically, a large-bore hypodermic needle is used to access the patient's vein or artery. Flashback of venous or arterial blood through the needle indicates to the physician when the tip of the needle is in the lumen of the blood vessel. A catheter guide is then inserted through the needle into the lumen of the blood vessel and the needle is withdrawn. A catheter is then inserted coaxially over the catheter guide into the lumen of the blood vessel. At this time the catheter guide may be withdrawn, leaving the catheter within the lumen of the blood vessel.

The construction of the catheter guide is critical to the successful completion of the Seldinger technique. The catheter guide must be flexible enough to exit the hypodermic needle and make the sometimes sharp turn into the vessel lumen without damaging or passing through the opposite wall of the vessel. The catheter guide must also be flexible enough to follow any sharp bends or tortuosity in the vessel without damaging the vessel wall. However, at the same time the catheter guide must be rigid enough to guide the catheter through the tissue and the vessel wall into the vessel lumen and to guide the catheter through any sharp bends, tortuosity or narrowing in the vessel without pulling back or kinking. These two requirements can sometimes be incompatible, particularly in difficult to catheterize vessels. For example, in placing venous catheters in neonates and premature infants, the catheter guide must be extremely flexible in order to avoid damaging the delicate vessel walls during insertion. However, such a flexible catheter guide may not be rigid enough to guide the catheter through the tissue and any sharp bends, tortuosity or narrowing in the vessel without pulling back or kinking. This can be extremely problematic in cases where successful catheterization is critical to the survival of the patient. It would be highly desirable in these cases to have a catheter guide that can be inserted into the blood vessel in a flexible state, and then can be rigidified to facilitate insertion of the catheter.

Typical prior art catheter guides are constructed with a coiled wire spring surrounding a core wire. This type of catheter guide is sometimes referred to in the literature as a guidewire or spring guide. Often the core wire is ground with a taper to provide the catheter guide with a flexible tip portion and a more rigid body portion. For most cases, this tapered core construction is adequate for providing the necessary combination of flexibility and stiffness. However, it necessarily involves a compromise in the characteristics of the catheter guide that will not be adequate in all cases, particularly in difficult cases like those described above.

At least one kind of variable stiffness catheter guide has been proposed in the prior art. These are known as movable core guidewires because they are constructed with a core wire that, instead of being welded in place within the guidewire, is slidable axially within the outer wire coil. This allows the core wire to be withdrawn so that the tip portion of the guidewire can be changed from stiff to flexible. However, the instructions for use provided with these products warn against advancing the movable core again once the guidewire has been inserted into the patient because of the danger that the core may exit the guidewire between the coils of the spring and damage or pierce the vessel wall. For this reason, such movable core guidewires do not provide an adequate means for changing a catheter guide from flexible to stiff after it has been inserted into the patient's blood vessel.

Flow-directed catheters are known in the prior art. These catheters generally have a very flexible catheter shaft with an inflatable balloon or other bulbous structure near the distal end, which is carried along by the blood flow in an artery or vein. For example, the SWANN-GANZ thermodilution catheter, manufactured by Edwards Laboratories, is a flow-directed catheter used for measuring cardiac output and pulmonary wedge pressure. The flexible catheter shaft is inserted into a patient through the jugular vein or other venous access site, then a small balloon on the distal end of the flexible catheter shaft is inflated with CO₂ and venous blood flow carries the inflated balloon through the right side of the heart and info the pulmonary artery. Another example of a flow-directed catheter is the MAGIC catheter manufactured by BALT Extrusions of France. This catheter is constructed with a catheter shaft having an extremely flexible distal section with a small bulbous structure molded on the distal end of the flexible distal section. This construction allows the flow-directed catheter to seek out high flow arterio-venous fistulas or shunts in the brain or elsewhere in the body by following the arterial blood flow. These flow-directed catheters however are not suitable as catheter guides. Furthermore, prior to the present invention, no one has suggested the use of a flow-directed catheter or catheter guide with a variable rigidity shaft that can be selectively changed between a flexible state and a rigid state.

### SUMMARY OF THE INVENTION

In keeping with the foregoing discussion, the present invention provides a catheter guide with a variable rigidity shaft. The variable rigidity shaft of the catheter guide can be selectively changed between a flexible state and a rigid state. The catheter guide can be inserted into a patient's vein or artery with the variable rigidity shaft in a flexible state to avoid trauma to the vessel walls. Once the catheter guide has been inserted, the variable rigidity shaft can be converted to the rigid state to provide a firm support for insertion of a catheter coaxially over the catheter guide. After the catheter has been inserted, the variable rigidity shaft is allowed to return to the flexible state to facilitate withdrawal of the catheter guide.

An additional feature of the invention is to provide a flow-directed catheter guide. The variable rigidity shaft of the catheter guide has on its distal end a deployable flow-directed member. After the catheter guide has been inserted into the patient's vein or artery with the variable rigidity shaft in the flexible state, the flow-directed member can be deployed to direct the distal end of the catheter guide downstream following the blood flow in the vessel. Generally, the flow-directed member keeps the catheter guide in the middle of the lumen where the velocity of the blood flow is greatest. Once the distal end of the catheter guide has reached the intended site or advanced to a predetermined depth, the flow-directed member can be retracted. Then, the variable rigidity shaft can be converted to the rigid state to provide support for insertion of a catheter coaxially over the catheter guide, as described above. After the catheter has been inserted, the variable rigidity shaft is allowed to return to the flexible state to facilitate withdrawal of the catheter guide.

In a further feature of the present invention, the flow-directed catheter guide with variable rigidity shaft may be provided as part of a kit for performing venous or arterial catheterization using a modified Seldinger technique or other technique.

### BRIEF DESCRIPTION OF THE DRAWINGS

FIG 1 shows a flow-directed catheter guide with variable rigidity shaft constructed according to the present invention with the flow-directed member in a deployed state.
FIG 2 is a phantom view of the distal end of the catheter guide of FIG 1 showing the flow-directed member in a folded and retracted state.
FIG 3 shows the distal end of the catheter guide of FIG 1 with the flow-directed member in a deployed state.
FIG 4 is a phantom view of the catheter guide of FIG 1 showing the construction of the variable stiffness shaft.
FIG 5 shows an alternate construction of the variable stiffness shaft.
FIG 6 shows another alternate construction of the variable stiffness shaft.
FIG 7 shows the flow-directed catheter guide with variable rigidity shaft of the present invention being deployed within a patient's blood vessel.
FIG 8 shows a variant of the flow-directed catheter guide with variable rigidity shaft of the present invention being deployed within a patient's blood vessel.

### DETAILED DESCRIPTION OF THE INVENTION

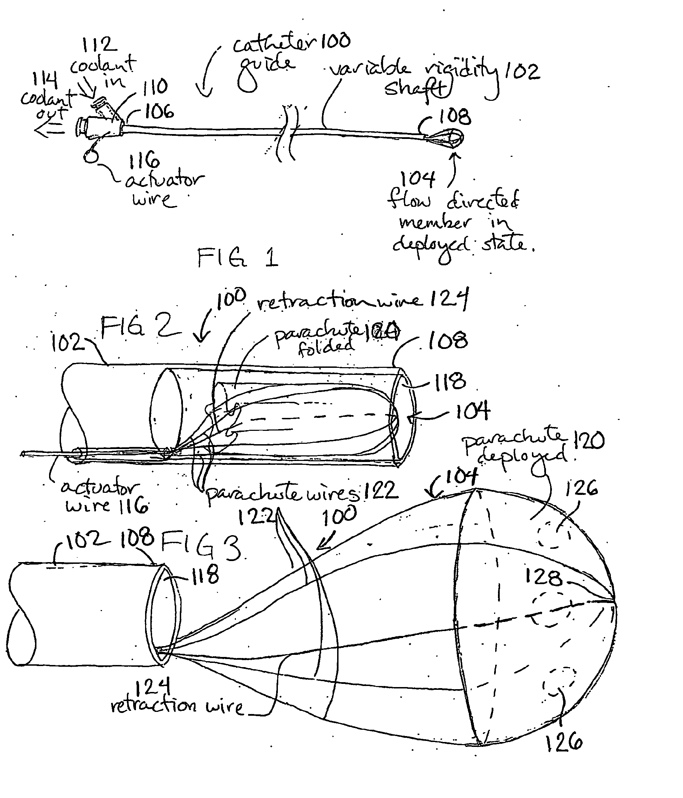

FIG 1 shows a flow-directed catheter guide 100 with a variable rigidity shaft 102 constructed according to the present invention. The catheter guide 100 has an elongated shaft 102 with a proximal end 106 and a distal end 108. At least a distal portion of the catheter guide shaft 102 is constructed as a variable rigidity shaft. The distal end 108 of the catheter guide 100 has a flow-directed member 104, which is shown in a deployed state. The proximal end 106 of the catheter guide 100 has a proximal fitting 110 with connections for inflow 112 and outflow 114 of coolant and an actuation wire 116 or the like for selectively actuating the flow-directed member 104. Optionally, the proximal fitting 110 may be removable from the elongated shaft 102.

Alternatively, the flow-directed member 104 described herein may be mounted on a catheter guide or catheter of more conventional construction. For example, the flow-directed member 104 may be mounted to the distal end of a conventional guidewire or spring guide with an elongated guide body that is constructed with a tapered core wire to provide a very flexible tip and gradually increasing stiffness in the proximal direction.

FIG 2 is a phantom view of the distal end 108 of the catheter guide 100 of FIG 1 showing the flow-directed member 104 in a folded and retracted state. In a preferred embodiment, the flow-directed member 104 is a parachute-like member that can be folded and retracted into a chamber or capsule 118 on the distal end 108 of the elongated shaft 102 of the catheter guide 100. The flow-directed member 104 has a parachute shroud 120 made of a fabric, plastic film or other biocompatible material. A plurality of parachute wires or cords 122 are attached to the periphery of the parachute shroud 120. The actuation wire 116 is connected to the plurality of parachute wires 122. Optionally, the flow-directed member 104 may also include a retraction wire 124 for inverting and retracting the parachute shroud 120 of the flow-directed member 104 back into the chamber 118 on the distal end 108 of the elongated shaft 102. Alternatively, the catheter guide may be constructed without the chamber 118 on the distal end 108 of the elongated shaft 102 and the folded parachute shroud 120 may reside in the lumen of the needle until it is deployed.

FIG 3 shows the distal end 108 of the catheter guide 100 of FIG 1 with the flow-directed member 104 in a deployed state. The flow-directed member 104 may be deployed by advancing the actuation wire 116 from the proximal end 106 of the catheter guide shaft 102 to push the parachute shroud 120 out of the chamber 118 on the distal end 108 of the elongated shaft 102. Optionally, the parachute shroud 120 may include one or more perforations 126 to reduce the force exerted by the blood flow on the deployed flow-directed member 104. The parachute wires 122 are attached around the periphery of the parachute shroud 120. The optional retraction wire 124 is connected to the peak 128 of the parachute shroud 120 of the flow-directed member 104 for inverting and retracting the parachute shroud 120 back into the chamber 118 on the distal end 108 of the elongated shaft 102. Optionally, the parachute wires 122 can be configured to be selectively releasable from the periphery of the parachute shroud 120, thus allowing the blood flow to invert the parachute shroud 120 so that it can be easily retracted using the centrally attached retraction wire 128.

In one preferred embodiment of the catheter guide 100, the parachute shroud 120 of the flow-directed member 104 is formed so that is assumes an approximately hemispherical shape when deployed in the patient's blood vessel. Alternatively, the parachute shroud 120 may be a simple flat panel of fabric or plastic film. In an alternate embodiment of the catheter guide 100, a parachute shroud 120 of either geometry may be mounted directly to the elongated shaft 102 without any parachute wires 122 and with or without a retraction wire 128 attached to the parachute shroud 120. In other alternate embodiments of the catheter guide 100, the flow-directed member 104 may be in the form of an inflatable balloon or a bulbous structure on the elongated shaft 102 of the catheter guide 100.

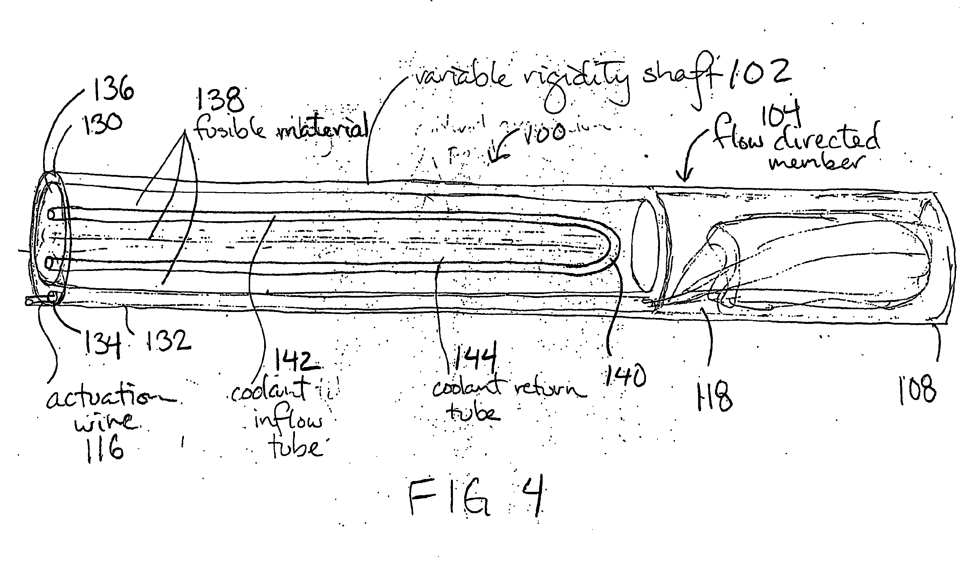

FIG 4 is a phantom view of the catheter guide 100 of FIG 1 showing the construction of the variable stiffness shaft 102. At least a distal portion of the catheter guide shaft 102 proximal to the chamber 118 for the flow-directed member 104 is constructed as a variable rigidity shaft. Optionally, the entire length of the catheter guide shaft 102 may be constructed as a variable rigidity shaft. The catheter guide shaft 102 is preferably constructed of a flexible polymer extrusion 130. The extrusion 130 has a sidewall 132 with an actuation lumen 134 for the actuation wire 116 extending within the sidewall 130 along the length of the catheter guide shaft 102 to the chamber 118 for the flow-directed member 104 at the distal end 108 of the shaft 102. The sidewall 132 encloses a main lumen 136 that contains a fusible material 138. A heat exchange tube 140 extends through the fusible material 138 in the main lumen 136 in a generally U-shaped configuration with a coolant inflow tube 142 and a coolant outflow tube 144. The fusible material 136 is preferably a fusible metal, or alternatively a fusible wax or polymer, with a melting point within a range from slightly below normal body temperature to slightly above normal body temperature.

If the fusible material 136 has a melting point slightly below normal body temperature, the variable rigidity shaft 102 is in the flexible state when it is at body temperature. If the melting point of the fusible material 136 is below room temperature, the variable rigidity shaft 102 will already be in the flexible state before it is inserted into the patient. If the melting point of the fusible material 136 is between room temperature and normal body temperature, the catheter guide 100 can be placed in a conditioning chamber to warm it to body temperature so that the variable rigidity shaft 102 is in the flexible state for insertion into the patient. The variable rigidity shaft 102 can be selectively rigidified by circulating a cooling fluid, either a liquid or gas, at a temperature below the melting temperature of the fusible material 136 through the heat exchange tube 140. The variable rigidity shaft 102 can be made more flexible again by circulating a warm fluid at a temperature above the melting temperature of the fusible material 136 through the heat exchange tube 140 or by simply allowing the temperature of the variable rigidity shaft 102 to equilibrate at body temperature.

If the fusible material 136 has a melting point slightly above normal body temperature, the variable rigidity shaft 102 is in the rigid state when it at body temperature. The variable rigidity shaft 102 can be converted to the flexible state for insertion into the patient by circulating a warm fluid at a temperature above the melting temperature of the fusible material 136 through the heat exchange tube 140 or by placing the catheter guide 100 in a conditioning chamber at a temperature above the melting temperature of the fusible material 136. The variable rigidity shaft 102 can be selectively rigidified by circulating a cooling fluid at a temperature below the melting temperature of the fusible material 136 through the heat exchange tube 140 or by simply allowing the temperature of the variable rigidity shaft 102 to equilibrate at body temperature. The variable rigidity shaft 102 can be made more flexible again by circulating a warm fluid at a temperature above the melting temperature of the fusible material 136 through the heat exchange tube 140.

If the fusible material 136 has a melting point at approximately normal body temperature, the variable rigidity shaft 102 can be converted to the flexible state for insertion into the patient by circulating a warm fluid at a temperature above the melting temperature of the fusible material 136 through the heat exchange tube 140 or by placing the catheter guide 100 in a conditioning chamber ai a temperature above the melting temperature of the fusible material 136. The variable rigidity shaft 102 can be selectively rigidified by circulating a cooling fluid at a temperature below the melting temperature of the fusible material 136 through the heat exchange tube 140. The variable rigidity shaft 102 can be made more flexible again by circulating a warm fluid at a temperature above the melting temperature of the fusible material 136 through the heat exchange tube 140.

Suitable materials for the fusible material 136 include, but are not limited to, the following materials, which are available from Indium Corp. (www.indium.com) as well as other suppliers:

**TABLE 1**

| | | | | | | |
|---|---|---|---|---|---|---|
| **Indalloy Number** | **Type** | **Liquidus C** | **Solidus C** | **Composition** | **Density lb/in³** | Specific **Gravity gm/cm³** |
| 60 | eutectic alloy | 15.7 | 15.7 | 75.5Ga/24.5ln | 0.2294 | 6.35 |
| 77 | ordinary alloy | 25.0 | 15.7 | 95Ga/5ln | 0.2220 | 6.15 |
| 14 | pure metal | 29.78 | 29.78 | 100Ga | 0.2131 | 5.904 |
| 117 | eutectic alloy | 47.2 | 47.2 | 44.7Bi 22.6Pb 19.1ln 8.3Sn 5.3Cd | | |

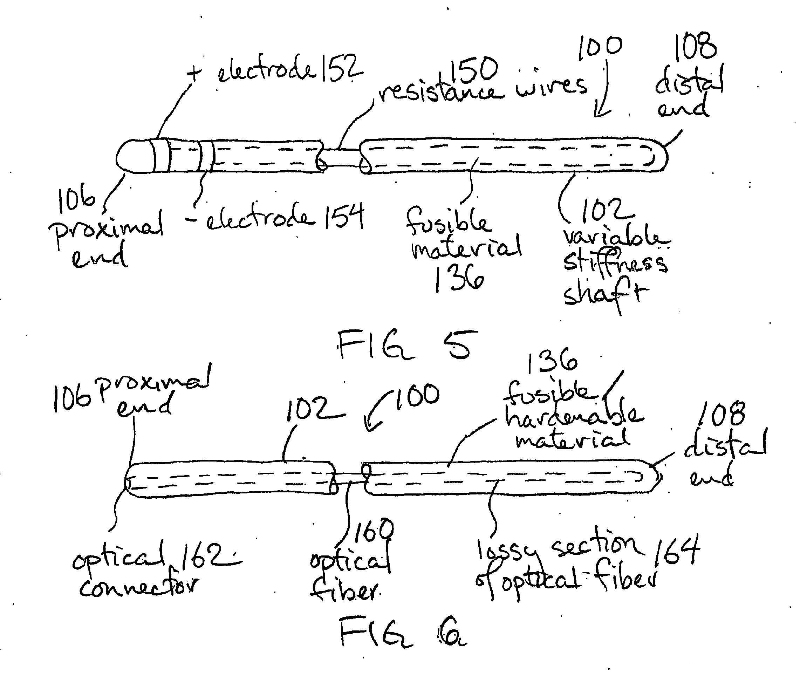

FIG 5 shows an alternate construction of the variable stiffness shaft 102. A distal portion or the entire shaft 102 of the catheter guide 100 may be constructed as a variable rigidity shaft. In this embodiment, the fusible material 136 has a melting point slightly above normal body temperature so the variable rigidity shaft 102 is in the rigid state when it is at body temperature. Instead of a heat exchange tube, the variable stiffness shaft 102 includes resistance wires 150 that are connected to a positive (+) electrode 152 and a negative (-) electrode 154 on the proximal end 106 of the catheter guide shaft 102. The variable rigidity shaft 102 can be converted to the flexible state for insertion into the patient by connecting the electrodes 152, 154 to a source of direct or alternating current to heat the fusible material 136 above its melting point. The variable rigidity shaft 102 can be selectively rigidified by allowing the temperature of the variable rigidity shaft 102 to equilibrate at body temperature. The catheter guide 100 may or may not be provided with a flow-directed member 104, as described above.

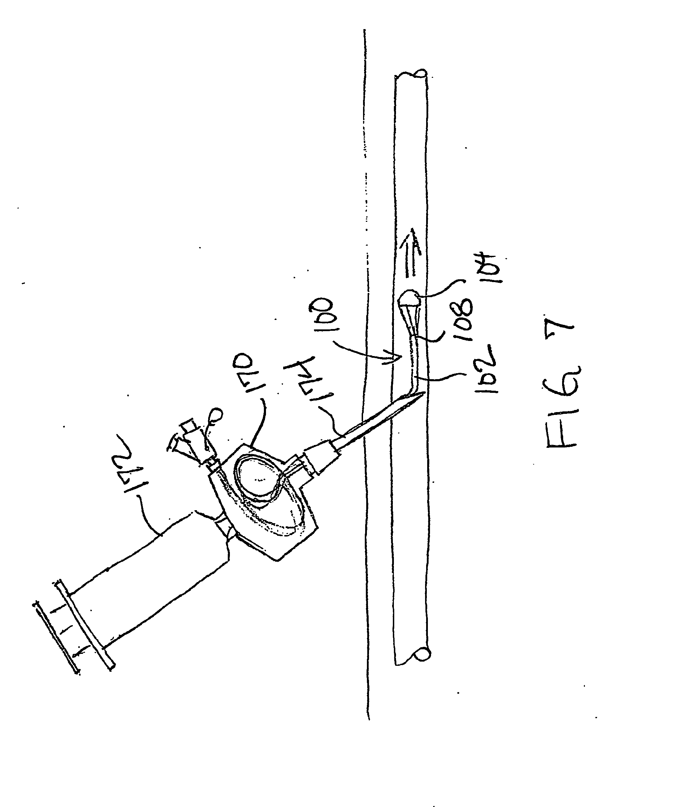

FIG 6 shows another alternate construction of the variable stiffness shaft 102. A distal portion or the entire shaft 102 of the catheter guide 100 may be constructed as a variable rigidity shaft. In this embodiment, the fusible material 136 has a melting point slightly above normal body temperature so the variable rigidity shaft 102 is in the rigid state when it at body temperature. Instead of a heat exchange tube, the variable stiffness shaft includes an optical fiber 160 connected to an optical connector 162 on the proximal end 106 of the catheter guide shaft 102. The optical fiber 160 has a lossy section 164 that extends the length of the variable stiffness shaft portion of the catheter guide shaft 102. The lossy section 164 can be created by removing the cladding from the optical fiber 160, by abrading the surface of the fiber and/or by creating microbends in the fiber 160. The variable rigidity shaft 102 can be converted to the flexible state for insertion into the patient by connecting the optical connector 162 to a source of intense light to heat the fusible material 136 above its melting point. The variable rigidity shaft 102 can be selectively rigidified by allowing the temperature of the variable rigidity shaft 102 to equilibrate at body temperature. The catheter guide 100 may or may not be provided with a flow-directed member 104, as described above.

Alternatively, the fusible material 136 in the embodiment of FIG 6 may be replaced with a hardenable material, such as an adhesive or a liquid polymer, that is hardened by exposure to visible or ultraviolet light. The variable rigidity shaft 102 is in the flexible state before it is inserted into the patient. The variable rigidity shaft 102 can be selectively rigidified by connecting the optical connector 162 to a source of visible or ultraviolet light to solidify the hardenable material. In this embodiment, the variable rigidity shaft 102 cannot be returned to the flexible state. However, the shaft 102 of the catheter guide 100 can be simply withdrawn from the patient in the rigid state without damage to the blood vessels. In another alternative embodiment of the catheter guide 100 of FIG 5 or FIG 6, the fusible material 136 in the variable rigidity shaft 102 may be replaced with a hardenable material, such as an adhesive or a liquid polymer, that is hardened by exposure to heat. The variable rigidity shaft 102 can be selectively rigidified by connecting the electrodes 152, 154 to a source of direct or alternating current or by connecting the optical connector 162 to a source of visible, infrared or ultraviolet light to heat the hardenable material to solidify it. In another alternative embodiment of the catheter guide 100, the fusible material 136 in the variable rigidity shaft 102 may be replaced with a hardenable material, such as an adhesive or a liquid polymer, that is hardened by exposure to other types of energy, such as ultrasonic vibrations, electromagnetic radiation or microwaves. The variable rigidity shaft 102 can be selectively rigidified by exposing the variable rigidity shaft 102 to the appropriate type of energy to solidify the hardenable material.

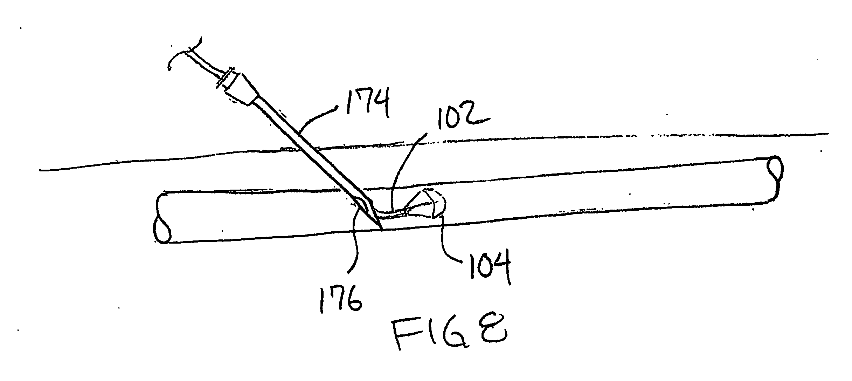

FIG 7 shows the flow-directed catheter guide 100 with variable rigidity shaft 102 of the present invention being deployed within a patient's blood vessel. The catheter guide 100 may be enclosed within an introduction chamber 170 attached between a syringe 172 and an introducer needle 174. This arrangement does not interfere with the use of needle safety devices, which are now required by regulations in some localities. The introducer needle 174 is inserted percutaneously into the patient's vein or artery. Flashback of venous or arterial blood through the needle 174 indicates to the physician when the tip of the needle 174 is in the lumen of the blood vessel. The catheter guide 100 is then inserted through the needle 174 into the lumen of the blood vessel with the variable rigidity shaft 102 in the flexible state. After the catheter guide 100 has been inserted into the patient's vein or artery with the variable rigidity shaft 102 in the flexible state, the flow-directed member 104 can be deployed to direct the distal end 108 of the catheter guide 100 downstream following the blood flow in the vessel. Once the distal end 108 of the catheter guide 100 has reached the intended site or advanced to a predetermined depth, the flow-directed member 104 can be retracted. Then, the variable rigidity shaft 102 can be converted to the rigid state to provide support for insertion of a catheter coaxially over the catheter guide 100. After the catheter has been inserted, the variable rigidity shaft 102 is allowed to return to the flexible state to facilitate withdrawal of the catheter guide 100.

FIG 8 shows a variant of the flow-directed catheter guide 100 with variable rigidity shaft 102 of the present invention being deployed within a patient's blood vessel. In this variant of the catheter guide 100, the introducer needle 174 has a flow window 176 on the upstream side of the needle 174. Initially, the flow-directed member 104 is positioned within the introducer needle 174 distal to the flow window 176. When the tip of the introducer needle 174 is in the lumen of the blood vessel, the blood flow enters the flow window 176 and catches the flow-directed member 104 and draws it out of the needle 174 into the lumen of the blood vessel. Once the flow-directed member 104 is in the lumen of the blood vessel, the flow-directed member 104 can deploy completely. Thus, the flow-directed catheter guide 100 is deployed automatically when the tip of the needle 174 is in the lumen of the blood vessel. The remainder of the method is performed as described above.

The variable stiffness shaft and the flow-directed aspects of the invention described above may be used separately or together for introduction of a catheter guide into both venous and arterial sites for various applications. Alternatively, the variable stiffness shaft and the flow-directed aspects of the invention may be adapted for use in a diagnostic or therapeutic catheter device. Some of the potential applications include insertion and placement of central venous lines, peripheral venous lines, peripherally inserted central (PIC) lines, SWANN-GANZ catheters, and therapeutic catheters, such as angioplasty or stenting catheters and therapeutic embolization catheters for treating aneurisms and arterio-venous fistulas or shunts.

## Claims

1. A catheter guide (100), comprising a deployable flow-directed member (104) located at a distal end (108) of the elongated shaft (102), **characterized by** :
an elongated shaft (102), wherein at least a portion of the elongated shaft contains a fusible material (138) and means (140; 150; 160) for selectively changing the temperature of the fusible material to provide selectively variable rigidity.

2. The catheter guide of claim 1, wherein the portion of the elongated shaft configured to provide selectively variable rigidity has a flexible state and a rigidified state, such that, when the portion of the elongated shaft is in the flexible state and the flow-directed member is deployed, the catheter guide is operable in a flow-directed mode within a patient's blood vessel and, when the portion of the elongated shaft is in the rigidified state, the catheter guide provides support for advancing a catheter over the catheter guide into the patient's blood vessel.

3. The catheter guide of claim 1, wherein the fusible material (138) has a melting temperature below normal human body temperature.

4. The catheter guide of claim 1, wherein the means for selectively changing the temperature of the fusible material (138) comprises a heat exchange tube (140) extending within the elongated shaft (102) and in thermal contact with the fusible material.

5. The catheter guide of claim 1, wherein the fusible material (138) has a melting temperature above normal human body temperature.

6. The catheter guide of claim 5, wherein the means for selectively changing the temperature of the fusible material (138) comprises a heat exchange tube (140) extending within the elongated shaft (102) and in thermal contact with the fusible material.

7. The catheter guide of claim 5, wherein the means for selectively changing the temperature of the fusible material (138) comprises an electrical resistance heater (150) extending within the elongated shaft (102) and in thermal contact with the fusible material.

8. The catheter guide of claim 5, wherein the means for selectively changing the temperature of the fusible material (138) comprises an optical fiber (160) extending within the elongated shaft (102) and in proximity with the fusible material.

9. The catheter guide of claim 1, wherein the deployable flow-directed member comprises a parachute-shaped member (104) selectively deployable from the distal end (108) of the elongated shaft (102).

10. The catheter guide of claim 9, wherein the parachute-shaped member has an undeployed position wherein the parachute-shaped member is contained within a chamber (118) connected with the distal end (108) of the elongated shaft (102).

11. The catheter guide of claim 10, further comprising a retraction member (124) connected to the parachute-shaped member (104) for retracting the parachute-shaped member (104) into the chamber (118).

## Patentansprüche

1. Katheterführung (100) mit einem entfaltbaren, in Strömungsrichtung befindlichen Element (104), das an einem distalen Ende (108) eines langgestreckten Schafts (102) angeordnet ist, **gekennzeichnet durch**:
einen langgestreckten Schaft (102), wobei mindestens ein Abschnitt des langgestreckten Schafts ein schmelzbares Material (138) und ein Mittel (140; 150; 160) zum wahlweisen Ändern der Temperatur des schmelzbaren Materials enthält, um wahlweise eine veränderliche Steifigkeit bereitzustellen.

2. Katheterführung nach Anspruch 1, wobei der Abschnitt des langgestreckten Schafts, der zur Bereitstellung der veränderlichen Steifigkeit konfiguriert ist, einen flexiblen Zustand und einen versteiften Zustand derart aufweist, daß, wenn der Abschnitt des langgestreckten Schafts sich im flexiblem Zustand befindet das in Strömungsrichtung befindliche Element entfaltet ist, die Katheterführung in einer in Strömungsrichtung gerichteten Weise innerhalb des Blutgefäßes eines Patienten betätigbar ist und daß, wenn sich der Abschnitt des langgestreckten Schafts in dem versteiften Zustand befindet, die Katheterführung für eine Abstützung zum Vorschieben eines Katheters über die Katheterführung in das Blutgefäß des Patienten sorgt.

3. Katheterführung nach Anspruch 1, wobei das schmelzbare Material (138) eine Schmelztemperatur unter der normalen Temperatur des menschlichen Körpers aufweist.

4. Katheterführung nach Anspruch 1, wobei das Mittel zum wahlweisen Ändern der Temperatur des schmelzbaren Materials (138) ein Wärmeaustauschrohr (140) umfaßt, das sich innerhalb des langgestreckten Schafts (102) erstreckt und in thermischem Kontakt mit dem schmelzbaren Material (138) steht.

5. Katheterführung nach Anspruch 1, wobei das schmelzbare Material (138) eine Schmelztemperatur über der normalen Temperatur des menschlichen Körpers aufweist.

6. Katheterführung nach Anspruch 5, wobei das Mittel zum wahlweisen Ändern der Temperatur des schmelzbaren Materials (138) ein Wärmeaustauschrohr (140) umfaßt, das sich innerhalb des langgestreckten Schafts (102) erstreckt und in thermischem Kontakt mit dem schmelzbaren Material (138) steht.

7. Katheterführung nach Anspruch 5, wobei das Mittel zum wahlweisen Ändern der Temperatur des schmelzbaren Materials (138) ein elektrisches Widerstandsheizelement (150) umfaßt, das sich innerhalb des langgestreckten Schafts (102) erstreckt und in thermischem Kontakt mit dem schmelzbaren Material (138) steht.

8. Katheterführung nach Anspruch 5, wobei das Mittel zum wahlweisen Ändern der Temperatur des schmelzbaren Materials (138) eine Lichtleitfaser (160) umfaßt, die innerhalb des langgestreckten Schafts (102) und in der Nähe des schmelzbaren Materials verläuft.

9. Katheterführung nach Anspruch 1, wobei das entfaltbare, in Strömungsrichtung befindliche Element ein fallschirmförmiges Element (104) umfaßt, das wahlweise aus dem distalen Ende (108) des langgestreckten Schafts (102) heraus entfaltbar ist.

10. Katheterführung nach Anspruch 9, wobei das fallschirmförmige Element eine nicht entfaltete Position hat, in der das fallschirmförmige Element in einer mit dem distalen Ende (108) des langgestreckten Schafts (102) verbundenen Kammer (118) untergebracht ist.

11. Katheterführung nach Anspruch 10, außerdem mit einem Rückziehelement (124), das mit dem fallschirmförmigen Element (104) verbunden ist, um das fallschirmförmige Element (104) in die Kammer (118) zurückzuziehen.

## Revendications

1. Guide de cathéter (100) comprenant un membre déployable dirigé vers l'écoulement (104), situé à une extrémité distale (108) de la tige allongée (102), **caractérisé par** :
une tige allongée (102), où au moins une partie de la tige allongée contient une matière fusible (138) et un moyen (140 ; 150 ; 160) pour changer sélectivement la température de la matière fusible pour produire une rigidité sélectivement variable.

2. Guide de cathéter selon la revendication 1, où la partie de la tige allongée configurée pour produire une rigidité sélectivement variable a un état flexible et un état rigidifié de sorte que, quand la partie de la tige allongée est dans l'état flexible et le membre dirigé vers l'écoulement est déployé, le guide de cathéter peut être actionné dans un mode dirigé vers l'écoulement dans un vaisseau sanguin d'un patient et, quand la partie de la tige allongée est dans l'état rigidifié, le guide de cathéter fournit un support pour faire avancer un cathéter sur le guide de cathéter dans le vaisseau sanguin du patient.

3. Guide de cathéter selon la revendication 1, où la matière fusible (138) a une température de fusion inférieure à la température normale du corps humain.

4. Guide de cathéter selon la revendication 1, où le moyen pour changer sélectivement la température de la matière fusible (138) comprend un tube d'échange de chaleur (140) qui s'étend dans la tige allongée (102) et en contact thermique avec la matière fusible.

5. Guide de cathéter selon la revendication 1, où la matière fusible (138) a une température de fusion supérieure à la température normale du corps humain.

6. Guide de cathéter selon la revendication 5, où le moyen pour changer sélectivement la température de la matière fusible (138) comprend un tube d'échange de chaleur (140) qui s'étend dans la tige allongée (102) et en contact thermique avec la matière fusible.

7. Guide de cathéter selon la revendication 5, où le moyen pour changer sélectivement la température de la matière fusible (138) comprend un dispositif chauffant à résistance électrique (150) qui s'étend dans la tige allongée (102) et en contact thermique avec la matière fusible.

8. Guide de cathéter selon la revendication 5, où le moyen pour changer sélectivement la température de la matière fusible (138) comprend une fibre optique (160) qui s'étend dans la tige allongée (102) et à proximité de la matière fusible.

9. Guide de cathéter selon la revendication 1, où le membre dirigé vers l'écoulement déployable comprend un membre en forme de parachute (104) déployable sélectivement depuis l'extrémité distale (108) de la tige allongée (102).

10. Guide de cathéter selon la revendication 9, où le membre en forme de parachute a une position non déployée dans laquelle le membre en forme de parachute est contenu dans une chambre (118) reliée à l'extrémité distale (108) de la tige allongée (102).

11. Guide de cathéter selon la revendication 10, comprenant en outre un membre de rétraction (124) relié au membre en forme de parachute (104) pour rétracter le membre en forme de parachute (104) dans la chambre (118).
